# EUROPÄISCHE PATENTSCHRIFT

(11) **EP 2 776 820 B1**
(45) Veröffentlichungstag und Bekanntmachung des Hinweises auf die Patenterteilung: **16.01.2019**
(21) Anmeldenummer: 12770007.8
(22) Anmeldetag: 13.09.2012
(51) Int. Cl.: G01N 27/417, G01N 33/00

(54) **VERFAHREN ZUR KORREKTUR VON MESSWERTEN EINES SENSORELEMENTS**
METHOD FOR CORRECTING MEASURED VALUES FROM A SENSOR ELEMENT
PROCÉDÉ DE CORRECTION DES VALEURS DE MESURE D'UN ÉLÉMENT DE CAPTEUR

(30) Priorität: 11.11.2011 DE 102011086144
(43) Veröffentlichungstag der Anmeldung: 17.09.2014
(73) Patentinhaber: Robert Bosch GmbH, 70442 Stuttgart (DE)
(72) Erfinder: LEDERMANN, Bernhard, 70499 Stuttgart (DE); BELZNER, Ulrich, 71701 Schwieberdingen (DE); STEINERT, Thomas, 71384 Weinstadt (DE)
(86) Internationale Anmeldenummer: PCT/EP2012/067963
(87) Internationale Veröffentlichungsnummer: WO 2013/068157

(56) Entgegenhaltungen:
- DE-A1- 10 163 912
- DE-A1- 19 838 466
- DE-A1-102006 061 565
- DE-A1-102008 001 697

## Beschreibung

### Stand der Technik

Verfahren zur Korrektur eines Messwerts eines Sensorelements, wobei das Sensorelement während wiederholt durchgeführter Messperioden zur Durchführung unterschiedlicher Messungen und zur Einstellung unterschiedlicher Betriebszustände durch eine Schaltungsanordnung in aufeinanderfolgenden Schaltpositionen in einer vorgegebenen Reihenfolge periodisch wechselnd elektrisch beschaltet wird, wobei zumindest eine Messung zur Bestimmung des Messwerts innerhalb einer Messperiode an vorgegebenen Schaltpositionen wiederholt durchgeführt wird und dadurch Einzelmesswerte an den vorgegebenen Schaltpositionen bestimmt werden und wobei der Messwert aus den Einzelmesswerten bestimmt wird.

Sensorelemente sind im Allgemeinen durch ein externes Messsystem elektrisch beschaltet. Dabei ist es häufig vorgesehen, dass das Messsystem durch verschiedene Schaltzustände zwischen verschiedenen Messungen und Betriebszuständen des Sensorelements umschaltet. Die Umschaltung kann in einer vorgegebenen Reihenfolge periodisch erfolgen, so dass ein Schaltzustand immer an der gleichen Schaltposition oder an gleichen Schaltpositionen einer Messperiode eingestellt wird.

Im Idealfall beeinflussen sich die aufeinanderfolgenden Messungen und Betriebszustände nicht. Praktisch kann es jedoch vorkommen, dass eine Messung durch einen zuvor eingestellten Betriebszustand, beispielhaft in Folge eines Polarisationseffekts im Sensorelement, verfälscht wird. Die Verfälschung der Messung ist somit von dem zuvor eingestellten Betriebszustand, und damit von der Schaltposition an der die Messung innerhalb der Messperiode durchgeführt wird, abhängig.

Sensorelemente, an welchen solche zyklisch wechselnden Beschaltungen vorgesehen sind, sind beispielhaft Breitbandlambdasonden, wie sie zur Überwachung der Zusammensetzung des Abgases von Brennkraftmaschinen auf Einhaltung von Grenzwerten verwendet werden. Die korrekte Funktion solcher Abgassensoren und insbesondere auch deren Alterungsbeständigkeit hängen stark von deren elektronischer Beschaltung ab. Die Funktionsblöcke einer solchen Beschaltung sind beispielhaft in der Schrift DE 10 2006 061 565 A1 beschrieben.

In der Schrift DE 10 2008 001697 A1 der Anmelderin wird eine verbesserte Beschaltung beschrieben, die es erlaubt, zusätzlich zu dem Betrieb des Abgassensors Informationen über den Betriebszustand der dort als Abgassensor verwendeten Breitbandlambdasonde zu erfassen, zu speichern und über eine Digitalschnittstelle an eine übergeordnete Motorsteuerung weiterzugeben. Diese Anordnung ermöglicht eine Diagnose der Kabelverbindungen zwischen der Beschaltung und der Breitbandlambdasonde auf Kurzschluß und Unterbrechung sowie auf Einhaltung der an den Anschlüssen zulässigen Spannungen. Die Betriebsbereitschaft der Abgassonde kann detektiert werden und deren Elektrodenpolarisation und die Alterung können kontinuierlich überwacht werden. Zur Durchführung dieser Messungen und zur Einstellung der verschiedenen Betriebszustände wird die Breitbandlambdasonde in aufeinanderfolgenden Schaltzuständen der Steuerelektronik unterschiedlich elektrisch beschaltet und entsprechend unterschiedlich elektrisch beaufschlagt. Dabei kann es zu einer Beeinflussung von Messungen durch vorherige Schaltzustände kommen. Beispielhaft kann ein Schaltzustand zu einer unerwünschten Polarisation einer Nernstzelle der Breitbandlambdasonde führen, die in einem nachfolgenden Schaltzustand zu einer Verfälschung des Messwerts der Nernstspannung an der Nernstzelle führt. Werden die Schaltzustände periodisch angewählt, ist auch die Beeinflussung der Messwerte periodisch. Eine Korrektur solcher Messfehler erfolgt heute meist durch einen Tiefpass-Filter, wodurch jedoch die Signaldynamik vermindert wird.

Es ist Aufgabe der Erfindung, ein Verfahren bereitzustellen, welches eine Korrektur von Pumpstrom-Messwerten eines zyklisch wechselnd beschalteten Sensorelements ermöglicht.

### Offenbarung der Erfindung

Die Aufgabe der Erfindung wird dadurch gelöst, dass die Einzelmesswerte in Abhängigkeit von der jeweiligen Schaltposition korrigiert werden. Da die Umschaltung zwischen den verschiedenen Schaltzuständen periodisch erfolgt, ist auch die Beeinflussung der Messung periodisch und damit in gewisser Weise systematisch und deterministisch. Durch die Schaltposition innerhalb der Messperiode ist der der Einzelmessung vorangegangene Betriebszustand des Sensorelements und somit die Beeinflussung des Einzelmesswerts durch die vorangegangene Beschaltung bekannt. Entsprechend können die Einzelmesswerte in Abhängigkeit von der Schaltposition innerhalb der Messperiode korrigiert werden. Während die nach dem Stand der Technik zur Korrektur der Messwerte über alle Schaltpositionen verwendeten Tiefpassfilter zu einem Dynamikverlust führen können, tritt dies bei dem erfindungsgemäßen Verfahren nicht auf. Durch die Korrektur der Einzelmesswerte ist auch der aus den Einzelmesswerten bestimmte Messwert korrigiert.

Erfindungsgemäß ist hierfür vorgesehen, dass in einer ersten Phase ein Näherungswert für den Messwert ermittelt wird, dass in einer Lernphase die Abweichungen der Einzelmesswerte von dem Näherungswert in Abhängigkeit von den jeweiligen Schaltpositionen innerhalb der Messperiode bestimmt werden, dass aus den Abweichungen Korrekturwerte zur Korrektur der Einzelmesswerte für die verschiedenen Schaltpositionen bestimmt werden und dass in einer Anwendungsphase die Einzelmesswerte mit dem der jeweiligen Schaltposition zugeordneten Korrekturwert korrigiert werden. Die Korrekturwerte für die Einzelmesswerte werden so in der Lernphase in Abhängigkeit von der Schaltposition eingelernt und können beispielsweise in einem nicht flüchtigen Datenspeicher gespeichert werden, so dass sie bei einem erneuten Start der Brennkraftmaschine bereits zur Korrektur vorliegen. In der Anwendungsphase erfolgt dann die eigentliche Korrektur der Einzelmesswerte mit den für die jeweiligen Schaltpositionen eingelernten Korrekturwerten.

Voraussetzung für die Ermittlung der schaltpositionsabhängigen Korrekturwerte ist, dass für die Bestimmung der Abweichungen der Einzelmesswerte von dem Näherungswert des Messwertes dieser Näherungswert bekannt ist. Daher kann es vorgesehen sein, dass in der ersten Phase der Näherungswert als Mittelwert über die Einzelmesswerte während einer Messperiode oder über mehrere Messperioden gebildet wird.

Die Genauigkeit der Korrektur der Einzelmesswerte und somit des Messwertes kann dadurch verbessert werden, dass die in der Anwendungsphase für die jeweiligen Schaltpositionen verwendeten Korrekturwerte als Mittelwerte oder als gleitende Mittelwerte der in mehreren Messperioden für die jeweiligen Schaltpositionen bestimmten Korrekturwerte gebildet werden. Hierbei können die Mittelwertbildung oder die gleitende Mittelwertbildung in Form einer Tiefpassfilterung für jeden Korrekturwert einzeln ausgeführt werden.

Ist es vorgesehen, dass die erste Phase, die Lernphase und die Anwendungsphase zeitlich parallel ablaufen, so können die Korrekturwerte permanent angepasst werden. Für die Korrektur der Einzelmesswerte werden so stets aktualisierte Korrekturwerte verwendet.

Eine deutliche Verbesserung der Korrektur des Pumpstroms einer Breitbandlambdasonde lässt sich dadurch erreichen, dass der Pumpstrom einer Breitbandlambdasonde in Abhängigkeit von Betriebsparametern einer Brennkraftmaschine korrigiert wird und dass anschließend die Korrektur der Einzelmesswerte des Pumpstroms vorgenommen wird.

Eine Berücksichtigung der Druck- und damit Drehzahlabhängigkeit des Pumpstroms einer Breitbandlambdasonde führt zu einer deutlichen Glättung des Messsignals. Daher kann es vorgesehen sein, dass als Betriebsparameter der Brennkraftmaschine deren Drehzahl verwendet wird.

Der Pumpstrom für die Pumpzelle einer Breitbandlambdasonde wird so eingestellt, dass die an einer unbestromten zugehörigen Nernstzelle auftretende Nernstspannung einen Wert von 450mV einnimmt. Damit dieses Verfahren als Maß für das Verbrennungsluftverhältnis Lambda gelten kann, wird eine Luftreferenz durch unipolare Stromimpulse an der Nernstzelle erzeugt. Diese unipolaren Stromimpulse können jedoch eine unerwünschte Polarisation der Nernstzelle erzeugen, die die Nernstspannung der unbestromten Nernstzelle beeinflusst. Der Näherungswert für den Pumpstrom liegt besonders nahe am korrekten Wert, wenn in der ersten Phase bei der Bestimmung des Näherungswerts der Mittelwert über Einzelmesswerte während Schaltpositionen gebildet wird, bei denen in der vorherigen Schaltposition keine Polarisation der Nernstzelle zu erwarten ist.

Das Verfahren lässt sich zur Korrektur des Pumpstroms einer Breitbandlambdasonde zur Bestimmung des Verbrennungsluftverhältnisses Lambda im Abgas von Brennkraftmaschinen anwenden.

Die Erfindung wird im Folgenden anhand eines in den Figuren dargestellten Ausführungsbeispiels näher erläutert. Es zeigen:
Figur 1 ein Zeitdiagramm eines Pumpstroms einer Breitbandlambdasonde,
Figur 2 ein Zeitdiagramm des Pumpstroms in einem dynamischen Betriebsfall.

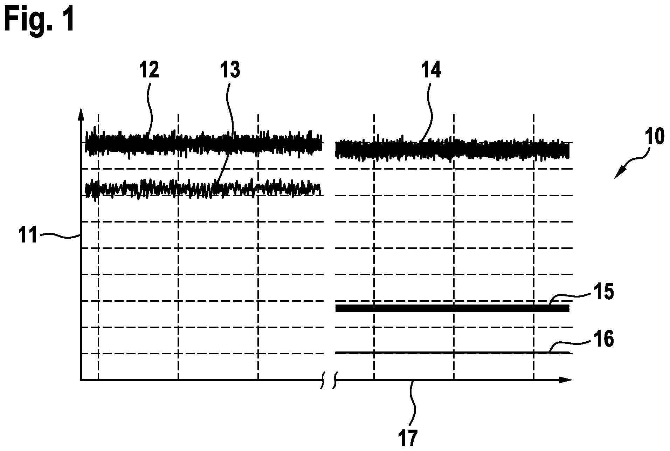

Figur 1 zeigt ein erstes Pumpstromdiagramm 10 mit einer ersten Signalachse 11 und einer ersten Zeitachse 17. In dem ersten Pumpstromdiagramm 10 sind Signale aus einem elektrischen Messsystem zum Betrieb einer Breitbandlambdasonde mit einer Pumpzelle und einer Nernstzelle dargestellt. In dem elektrischen Messsystem wird ein ASIC vom Typ CJ135 verwendet, das innerhalb einer Messperiode von 9,99 Millisekunden 15 unterschiedliche Schaltzustände einnimmt um Mess- und Diagnosefunktionen auszuführen. Wird das Messsystem ohne die erfindungsgemäße Korrekturfunktion betrieben, tritt während eines Messzyklus eine Gruppe von ersten Pumpstromwerten 12 und eine Gruppe von zweiten Pumpstromwerten 13 auf. Ein Grund für auftretenden Unterschiede zwischen den ersten Pumpstromwerten 12 und den zweiten Pumpstromwerten 13 ist die bei einigen Schaltzuständen des ASIC CJ135 auftretende Polarisation der Nernstzelle der Breitbandlambdasonde.

Erfindungsgemäß wird aus den ersten Pumpstromwerten 12 und den zweiten Pumpstromwerten 13 ein Näherungswert für den Pumpstrom gebildet. Aus den Abweichungen der ersten Pumpstromwerte 12 und der zweiten Pumpstromwerten 13 von dem Näherungswert werden Korrekturwerte gebildet, die in dem ersten Pumpstromdiagramm 10 als erste Korrekturwerte 15 und zweite Korrekturwerte 16 eingetragen sind. Insgesamt werden so 15 Korrekturwerte, für jeden Schaltzustand des ASIC CJ135 ein Korrekturwert, gebildet. Für jeden einzelnen der Korrekturwerte wird dabei mittels eines Tiefpaßfilters über mehrere Meßperioden ein Gleitmittel gebildet, das in einem nicht-flüchtigen Datenspeicher abgelegt wird. Die ersten Pumpstromwerte 12 und die zweiten Pumpstromwerte 13 werden mit den jeweils zugehörigen Werten aus den ersten Korrekturwerten 15 und den zweiten Korrekturwerten 16 korrigiert und es werden erfindungsgemäß die korrigierten Pumpstromwerte 14 gebildet.

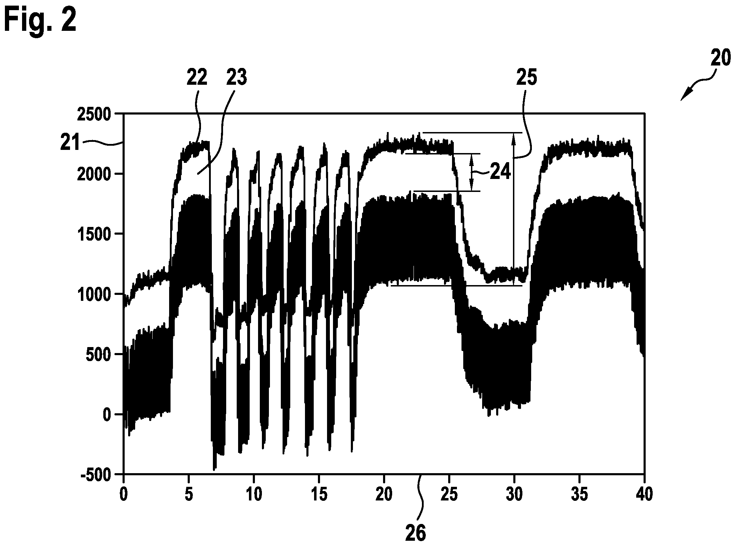

Figur 2 zeigt in einem zweiten Pumpstromdiagramm 20 einen Signalverlauf für einen dynamischen Betriebsfall einer Brennkraftmaschine. Entlang einer zweiten Zeitachse 26 sind auf einer zweiten Signalachse 21 ein erster Pumpstromverlauf 22 ohne Korrektur und ein zweiter Pumpstromverlauf 23 mit der erfindungsgemäßen Korrektur dargestellt. Der erste Pumpstromverlauf 22 überstreicht dabei in einem mittleren Bereich der zweiten Zeitachse 26 einen ersten Pumpstrombereich 25, der von den untersten dunkel dargestellten Werten bis zu den höchsten dunkel dargestellten Werten reicht. Durch Anwendung der erfindungsgemäßen Korrektur wird der erste Pumpstrombereich 25 auf einen zweiten Pumpstrombereich 24 beschränkt, dessen Werte im zweiten Pumpstromdiagramm 20 hell im Vordergrund der dunkel dargestellten Werte des ersten Pumpstrombereichs 25 überlagert sind. Es ist sichtbar, dass durch das erfindungsgemäße Verfahren die Genauigkeit bei der Bestimmung des erforderlichen Pumpstroms verbessert wird. Eine weiter Verbesserung des Verfahrens kann dadurch erreicht werden, dass berücksichtigt wird, dass Druckunterschiede im Abgaskanal der Brennkraftmaschine den Pumpstrom beeinflussen. Diese können durch eine Korrektur der Pumpstromwerte abhängig von der Drehzahl der Brennkraftmaschine berücksichtigt werden.

## Patentansprüche

1. Verfahren zur Korrektur eines Pumpstrom-Messwerts einer Breitbandlambdasonde, wobei die Sonde während wiederholt durchgeführter Messperioden zur Durchführung unterschiedlicher Messungen und zur Einstellung unterschiedlicher Betriebszustände durch eine Schaltungsanordnung in aufeinander folgenden Schaltpositionen in einer vorgegebenen Reihenfolge periodisch wechselnd elektrisch beschaltet wird, wobei zumindest eine Messung zur Bestimmung des Messwerts innerhalb einer Messperiode an vorgegebenen Schaltpositionen wiederholt durchgeführt wird und dadurch Einzelmesswerte (12,13) an den vorgegebenen Schaltpositionen bestimmt werden und wobei der Messwert aus den Einzelmesswerten bestimmt wird, **dadurch gekennzeichnet, dass** die Einzelmesswerte in Abhängigkeit von der jeweiligen Schaltposition korrigiert werden indem in einer ersten Phase ein Näherungswert für den Messwert ermittelt wird, in einer Lernphase die Abweichungen der Einzelmesswerte von dem Näherungswert in Abhängigkeit von der Schaltposition innerhalb der Messperiode bestimmt werden und aus den Abweichungen Korrekturwerte (15,16) zur Korrektur der Einzelmesswerte für die verschiedenen Schaltpositionen bestimmt werden und in einer Anwendungsphase die Einzelmesswerte (12,13) mit dem der jeweiligen Schaltposition zugeordneten Korrekturwert (15,16) korrigiert und dadurch der korrigierte Pumpstrom-Messwert (14) gebildet wird.

2. Verfahren nach Anspruch 1, **dadurch gekennzeichnet, dass** in der ersten Phase der Näherungswert als Mittelwert über die Einzelmesswerte während einer Messperiode oder über mehrere Messperioden gebildet wird.

3. Verfahren nach einem der Ansprüche 1 oder 2, **dadurch gekennzeichnet, dass** die in der Anwendungsphase für die jeweiligen Schaltpositionen verwendeten Korrekturwerte als Mittelwerte oder als gleitende Mittelwerte der in mehreren Messperioden für die jeweiligen Schaltpositionen bestimmten Korrekturwerte gebildet werden.

4. Verfahren nach einem der Ansprüche 1 bis 3, **dadurch gekennzeichnet, dass** die erste Phase, die Lernphase und die Anwendungsphase zeitlich parallel ablaufen.

5. Verfahren nach einem der Ansprüche 1 bis 4, **dadurch gekennzeichnet, dass** der Pumpstrom der Breitbandlambdasonde in Abhängigkeit von Betriebsparametern einer Brennkraftmaschine korrigiert wird und dass anschließend die Korrektur der Einzelmesswerte des Pumpstroms vorgenommen wird.

6. Verfahren nach Anspruch 5, **dadurch gekennzeichnet, dass** als Betriebsparameter der Brennkraftmaschine deren Drehzahl verwendet wird.

7. Anwendung des Verfahrens nach einem der vorgenannten Ansprüche zur Korrektur des Pumpstroms einer Breitbandlambdasonde zur Bestimmung des Verbrennungsluftverhältnisses Lambda im Abgas von Brennkraftmaschinen.

## Claims

1. Method for correcting a pump current measured value from a broadband lambda probe, wherein the probe is electrically connected in a periodically alternating manner in successive switching positions in a predefined order by a circuit arrangement during measurement periods which are repeatedly carried out for the purpose of carrying out different measurements and setting different operating states, wherein at least one measurement is repeatedly carried out for the purpose of determining the measured value within a measurement period at predefined switching positions and individual measured values (12, 13) at the predefined switching positions are determined thereby, and wherein the measured value is determined from the individual measured values, **characterized in that** the individual measured values are corrected on the basis of the respective switching position by virtue of the fact that an approximate value is determined for the measured value in a first phase, the deviations of the individual measured values from the approximate value are determined in a learning phase on the basis of the switching position within the measurement period and the deviations are used to determine correction values (15, 16) for correcting the individual measured values for the different switching positions, and the individual measured values (12, 13) are corrected in an application phase using the correction value (15, 16) assigned to the respective switching position and the corrected pump current measured value (14) is formed thereby.

2. Method according to Claim 1, **characterized in that** the approximate value is formed as an average value of the individual measured values during a measurement period or over a plurality of measurement periods in the first phase.

3. Method according to either of Claims 1 and 2, **characterized in that** the correction values used in the application phase for the respective switching positions are formed as average values or as sliding average values of the correction values determined in a plurality of measurement periods for the respective switching positions.

4. Method according to one of Claims 1 to 3, **characterized in that** the first phase, the learning phase and the application phase take place in a parallel manner in terms of time.

5. Method according to one of Claims 1 to 4, **characterized in that** the pump current of the broadband lambda probe is corrected on the basis of operating parameters of an internal combustion engine, and **in that** the individual measured values of the pump current are then corrected.

6. Method according to Claim 5, **characterized in that** the speed of the internal combustion engine is used as the operating parameter of the latter.

7. Use of the method according to one of the above-mentioned claims correcting the pump current of a broadband lambda probe for the purpose of determining the air-fuel ratio lambda in the exhaust gas of internal combustion engines.

## Revendications

1. Procédé de correction d'une valeur mesurée de courant de pompage d'une sonde lambda à large bande, la sonde étant connectée électriquement périodiquement en alternance pendant des périodes de mesure effectuée de manière répétée en vue de réaliser différentes mesures et en vue de régler des états opérationnels différents par un arrangement de commutation dans des positions de commutation successives dans un ordre prédéfini, au moins une mesure destinée à définir la valeur mesurée étant effectuée de manière répétée à l'intérieur d'une période de mesure à des positions de commutation prédéfinies et des valeurs mesurées individuelles (12, 13) étant ainsi définies aux positions de commutation prédéfinies et la valeur mesurée étant définie à partir des valeurs mesurées individuelles, **caractérisé en ce que** les valeurs mesurées individuelles sont corrigées en fonction de la position de commutation respective en déterminant, dans une première phase, une valeur approximative pour la valeur mesurée, en définissant, dans une phase d'apprentissage, les écarts entre les valeurs mesurées individuelles et la valeur approximative en fonction de la position de commutation à l'intérieur de la période de mesure et en définissant, à partir des écarts, des valeurs de correction (15, 16) destinées à corriger les valeurs mesurées individuelles pour les différentes positions de commutation et en corrigeant, dans une phase d'application, les valeurs mesurées individuelles (12, 13) avec la valeur de correction (15, 16) associée à la position de commutation respective et en formant ainsi la valeur mesurée de courant de pompage corrigée (14).

2. Procédé selon la revendication 1, **caractérisé en ce que** dans la première phase, la valeur approximative est formée en tant que valeur moyenne sur les valeurs mesurées individuelles pendant une période de mesure ou sur plusieurs périodes de mesure.

3. Procédé selon l'une des revendications 1 ou 2, **caractérisé en ce que** les valeurs de correction utilisées dans la phase d'application pour les positions de commutation respectives sont formées en tant que valeurs moyennes ou valeurs moyennes glissantes des valeurs de correction définies pour les positions de commutation respectives dans plusieurs périodes de mesure.

4. Procédé selon l'une des revendications 1 à 3, **caractérisé en ce que** la première phase, la phase d'apprentissage et la phase d'application se déroulent en parallèle dans le temps.

5. Procédé selon l'une des revendications 1 à 4, **caractérisé en ce que** le courant de pompage de la sonde lambda à large bande est corrigé en fonction des paramètres de fonctionnement d'un moteur à combustion interne et **en ce que** la correction des valeurs mesurées individuelles du courant de pompage est effectuée ensuite.

6. Procédé selon la revendication 5, **caractérisé en ce que** le paramètre de fonctionnement utilisé du moteur à combustion interne est sa vitesse de rotation.

7. Application du procédé selon l'une des revendications précédentes pour corriger le courant de pompage d'une sonde lambda à large bande en vue de définir le taux d'air de combustion lambda dans les gaz d'échappement de moteurs à combustion interne.
